(19) **Europäisches Patentamt / European Patent Office / Office européen des brevets**

(11) **EP 2 999 393 B1**

(12) **FASCICULE DE BREVET EUROPEEN**

(45) Date de publication et mention
de la délivrance du brevet:
**08.09.2021 Bulletin 2021/36**

(21) Numéro de dépôt: **14741572.3**

(22) Date de dépôt: **16.07.2014**

(51) Int Cl.:
***A61B 3/11*** *(2006.01)*   ***A61B 3/14*** *(2006.01)*

(86) Numéro de dépôt international:
**PCT/EP2014/065266**

(87) Numéro de publication internationale:
**WO 2015/007784 (22.01.2015 Gazette 2015/03)**

(54) **PROCÉDÉ DE DÉTERMINATION DE MESURES OCULAIRES AVEC UN CAPTEUR GRAND PUBLIC**

VERFAHREN ZUR BESTIMMUNG VON AUGENMESSUNGEN UNTER VERWENDUNG EINES VERBRAUCHERSENSORS

METHOD FOR DETERMINING OCULAR MEASUREMENTS USING A CONSUMER SENSOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**30.03.2016 Bulletin 2016/13**

(73) Titulaire: **Fittingbox**
**31670 Labège (FR)**

(72) Inventeurs:
• **CHOUKROUN, Ariel**
**31400 Toulouse (FR)**

• **LE GALLOU, Sylvain**
**F-31450 Baziege (FR)**

(74) Mandataire: **Ipside**
**6, Impasse Michel Labrousse**
**31100 Toulouse (FR)**

(56) Documents cités:
**EP-A1- 1 728 467    WO-A1-2011/113936**
**WO-A1-2012/041545    WO-A1-2013/045531**
**DE-A1- 10 207 316    US-A1- 2014 152 956**
**US-B1- 6 535 223**

**Description**

**Domaine de l'invention**

L'invention relève du domaine de l'optique. Elle concerne plus particulièrement le domaine de la fabrication de verres correcteurs pour la vue.

**Art antérieur**

Dans le cadre de la fabrication et du montage de verres de lunettes correcteurs, il est nécessaire de connaître avec le plus de précision possible des mesures oculaires telles que la distance pupillaire, la distance mono Pupillaire et les hauteurs.

La distance pupillaire (nommée **PD** dans la suite de la description) est la distance entre les centres des pupilles lorsque le sujet regarde à l'infini.

La distance mono pupillaire (notée **MonoPD** dans la suite de la description) est la distance entre la projection de la pupille dans le plan du verre et le centre de la monture. Cette grandeur mesure le décentrement horizontal à appliquer lors du taillage du verre.

Les hauteurs sont les distances entre la projection des pupilles dans le plan du verre et le bas de la monture (intérieur présumé de la dragée interne). Cette grandeur mesure le décentrement vertical à appliquer lors du taillage du verre. L'état le proche quant à la présente invention est représenté par le document US 6535223 qui divulgue un procédé standard de détermination de la distance pupillaire et des hauteurs et par le WO2012/04154995 qui décrit un procédé de prise et de fourniture d'une image du visage.

**Exposé de l'invention**

L'invention consiste à déterminer les mesures oculaires et optiques permettant la fabrication et le montage précis de verres correcteurs sur une paire de lunettes à partir d'une image.

L'apport de la présente_méthode est qu'elle garantit une mesure précise et fiable même si les caméras sont des caméras à forte ouverture de champ, comme des webcams ou des caméras mobiles, et que la qualité des images n'est pas bonne : résolution faible et compression de l'image forte. Les autres méthodes quant à elles fonctionnent en théorie mais ne garantissent pas la précision nécessaire en pratique.

Cette demande de brevet est une déclinaison de l'analyse en 3D proposée dans le brevet FR 10 52001, sur des mesures à partir d'une seule image. Le procédé décrit ici garantit une précision de 0.5mm, atteignable avec des caméras du marché, même de mauvaise qualité. Il est applicable en contexte 'internet', à la maison, avec des outils de tous les jours, et en magasin d'optique. Les solutions qui paraitraient évidentes à l'homme du métier sont analysées et leur invalidité théorique et pratique est expliquée.

Contrairement à l'art antérieur, nous proposons des solutions de mesures oculaires réalisables sans expert avec des capteurs grand public (donc dans tous les contextes) et ne nécessitant pas de disposer d'une mire complexe. La mire pourra être aussi bien un objet de taille connue se trouvant à disposition de l'utilisateur (carte de crédit par exemple) ou bien même son propre visage en 3D reconstruit métriquement. Nous proposons aussi un protocole particulier qui ne nécessite aucune mire dans la scène.

L'invention vise ainsi sous un premier aspect un procédé de détermination d'au moins une mesure oculaire (distance pupillaire, distance mono Pupillaire et / ou hauteurs) d'un utilisateur en utilisant un capteur d'images numériques, de type grand public. Le procédé utilise au moins une image de la tête de l'utilisateur, acquise par le capteur d'images, contenant un objet de taille connue, et les paramètres de calibrage de la caméra sont inconnus ou connus avec peu de précision.

La mesure est possible en une seule acquisition d'image, ou plusieurs, contrairement aux méthodes précédentes qui sont erronées avec une seule image, ou demandent un ensemble conséquent d'images pour proposer une solution fiable.

Dans des modes de réalisation :

- l'objet de taille connue est le visage de l'utilisateur.
- l'objet de taille connue est un objet plan.
- l'objet de taille connue est une paire de lunettes.

Dans un mode de réalisation particulier, la tête de l'utilisateur est disposée de face sur au moins une image.

Dans un mode de réalisation particulier, l'utilisateur est à une distance connue du dispositif d'acquisition et l'objet de taille connue est positionné à une distance connue de la zone faisant l'objet de la mesure oculaire.

Dans un mode de réalisation particulier, l'utilisateur est guidé par un système interactif d'aide au bon positionnement.

Dans un mode de réalisation particulier, le système interactif d'aide au bon positionnement est une forme dessinée sur l'écran d'affichage (une forme de visage par exemple).

Dans un mode de réalisation particulier, le système interactif d'aide au bon positionnement comporte des consignes issues d'un suivi en temps réel du visage de l'utilisateur.

Dans des modes de réalisation :

- le capteur d'images est calibré et acquiert des images en se déplaçant autour du visage de l'utilisateur qui regarde un point à l'infini.
- le capteur d'images est calibré et acquiert des images en se déplaçant autour du visage de l'utilisateur qui regarde des points affichés sur un écran.
- le capteur d'images est calibré et fournit une carte de profondeur de la scène.

Le capteur d'images est alors par exemple de type Kinect -marque déposée-.

### Brève description des figures

Les buts et avantages de l'invention seront mieux compris à la lecture de la description et des dessins d'un mode particulier de réalisation, donné à titre d'exemple non limitatif, et pour lequel les dessins représentent :

- figure 1 : une représentation schématique des paramètres pris en compte dans la mesure,
- figure 2 : une vue schématique d'une image obtenue par la caméra,
- figure 3 : un schéma des éléments intervenants dans la prise de mesures.

### Description détaillée d'un mode de réalisation de l'invention

### Report de mesure et systèmes existants : analyse

Afin d'obtenir la mesure d'une grandeur particulière (PD, monoPD, hauteur) à partir d'une image, nous proposons d'effectuer un report de mesure. En effet, connaissant la taille réelle d'un objet présent dans l'image, il est possible d'obtenir une estimation de la taille réelle de la grandeur souhaitée en comparant leur taille en pixel dans l'image. Contrairement aux systèmes existants n'imposant pas de contraintes sur le regard du sujet et sur la façon de positionner l'objet mire, notre procédé permet d'utiliser le report de mesure de manière optimale en contrôlant ainsi la précision de la mesure.

En effet, l'ensemble des protocoles proposés sont soumis à des erreurs de parallaxe et de reports induites par la distance entre l'objet de référence et l'objet à mesurer, à savoir les mesures de distance oculaire, ainsi que l'orientation des plans contenant objets de référence et objet de mesure. Bien qu'en théorie il soit possible d'effectuer un report de mesure de deux objets dans une image, la précision nécessaire pour les mesures oculaires n'est jamais garantie lors de la réalisation du protocole, du fait de contraintes théoriques et pratiques non respectées.

Nous listons ici les cas d'erreur pour une mesure à partir d'une seule image et d'une caméra quelconque. Ces cas d'erreur sont communément commis dans toutes les techniques de reports de mesure existantes à partir d'un objet connu.

Au niveau du protocole de réalisation, nous considérons que l'utilisateur positionne une mire (objet de dimensions connues) planaire en l'appuyant contre son visage, centré au niveau des yeux, c'est-à-dire sur la bouche ou sur le front, afin de former un système rigide visage/mire.

Dans le cas d'une image fixe (issue d'une photographie, vidéo ou flux temps-réel), nous considérons inconnus les paramètres intrinsèques de la caméra. Si nous considérons un modèle en trou d'épingle, le paramètre le plus influent pour les erreurs est la distance focale, et dans une moindre mesure pour les caméras actuelles, la position du centre optique et les distorsions radiales. Lors de la prise de vue, des erreurs de réalisation pratiques, qui influent sur les valeurs et les confiances de ces paramètres, peuvent survenir et se combiner entres elles, mais aussi avec les imprécisions des paramètres de la numérisation de l'image : résolution, qualité et dynamique (compensations, etc.) du capteur, compression de l'image.

Pour les paramètres liés au modèle de caméra et à la scène, et pour un report de mesure 2D dans le plan image, entre la mire de taille connue, et la grandeur à mesurer, la distance entre les centres des pupilles, on note les configurations suivantes :

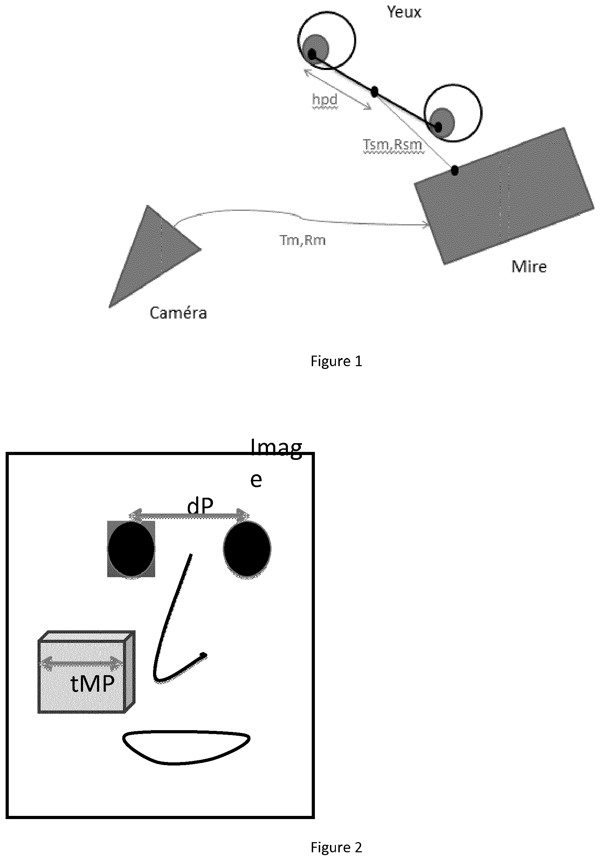

**Relation du système rigide utilisateur/mire** (noté dans la suite UM), **par rapport à la caméra** (voir figure 1)

∘ Position :

- Distance : plus le système Utilisateur / Mire (UM) est proche de la caméra, moins les relations de distances entre les points visibles de la mire et du visage sont conservées entre l'image et la scène 3D. Ces relations et points du système utilisateur / mire UM en 3D peuvent même disparaître dans l'image en fonction de la distance à la caméra, comme par exemple les oreilles qui disparaissent du champ de visibilité lorsqu'on s'approche très près. Plus le système utilisateur / mire UM est loin de la caméra, plus l'on se rapproche d'un modèle de projection orthographique, c'est-à-dire que les relations de mesure visibles sur l'image sont conservées par rapport à la réalité. Cela est vrai pour tous les points qui ne sont pas situés sur un plan fronto-parallèle au plan image de la caméra.
- Position dans l'image : plus le système utilisateur / mire UM est aligné avec l'axe optique, et donc centré dans l'image pour la majorité des caméras, moins il y a d'erreur sur la mesure. Si les plans mire et mesure à effectuer (distance pupillaire) sont confondus, le report de mesure peut comporter un biais si les grandeurs à mesurer ne sont pas centrées. Lorsqu'on effectue un report de mesure pour une grandeur horizontale et que le sujet est bien centré dans l'image, alors l'erreur de report de distances est centrée, et ne dépend plus que de la distance entre les plans de mesure et de référence si ces plans sont fronto-parallèles à la caméra.

  Ainsi, pour effectuer un report de mesure, le système utilisateur / mire UM doit se situer loin de la caméra (appelée faible perspective, proche de la vue orthographique) et au centre de l'image. Il doit aussi être symétrique et centré entre les grandeurs à mesurer (distance entre les yeux) et les grandeurs de référence (objet mire) selon les dimensions nécessaires à la mesure.

∘ Orientation : dans le cas où le système est orienté par rapport à la caméra (visage tourne vers la droite ou la gauche, ou haut ou bas), il n'est possible d'effectuer un report de mesure qu'on appellera suffisant que dans le cas où grandeur de référence et grandeur à mesurer unidimensionnelle sont sur le même plan, centrées, de taille comparable, et d'orientation faible. Sinon, en théorie (voir par exemple : "Multiple View Geometry") les transformations projectives ne conservant que le birapport, il faut une relation particulière entre 8 points de la scène pour pouvoir effectuer le report de mesure. Cette configuration est possible et amène à une interprétation métrique 3D. Par contre, pour un report de mesure 2D sur l'image, les erreurs vont grandir très vite avec l'orientation, d'autant plus vite que les grandeurs à mesurer sont décentrées.

**Relation Objet mire, caméra**
Les conclusions sont ici les mêmes que ci-dessus.

**Relation Objet mire, visage**

∘ Position profondeur : si l'objet mire est dans le même plan que la grandeur à mesurer, alors, un report peut être fait. Si les plans sont décalés en profondeur par rapport à la caméra, l'erreur de report de mesure commise va dépendre de la relation entre la distance caméra et la distance Mire/Utilisateur. Plus le couple utilisateur / mire UM est loin de la caméra, plus l'erreur de report due au décalage de profondeur est petite. Plus le couple utilisateur / mire UM est près, plus l'erreur de mesure est importante au regard de la précision attendue.

∘ Position et éloignement : plus l'objet mire est éloigné des yeux, plus l'erreur sur le report est grande. En effet, les erreurs de parallaxe dues à la position dans l'image interviennent dans la mesure elles-mêmes de chaque entité et faussent le rapport entre les objets mire et yeux. Si l'on considère que le visage est situé au centre de l'image le long d'un axe de symétrie vertical passant par le nez, alors on positionnera aussi la mire telle que la mesure soit répartie équitablement de part et d'autre de la projection du centre optique dans l'image, afin de limiter les erreurs de parallaxe intrinsèques à la position de la mire et relativement à la grandeur à mesurer sur le visage.

∘ Orientation : l'orientation apporte aussi le problème de parallaxe vu plus haut.

∘ Forme de l'objet : l'objet doit être petit (autour d'une dizaine de centimètres) et plan, afin de tenir le stablement possible sur la bouche et le menton, ou sur le front. Des objets mire trop grands, lourds ou épais ne permettent pas une telle stabilité. Un Smartphone qui afficherait une image plane peut être utilisé, mais son poids comparativement à une carte de crédit ou un CD (Compact Disk) est plus important et apporte moins de confort de positionnement.

∘ Contact rigide : si l'objet mire n'est pas en contact rigide avec le visage, il n'y a aucune garantie pour le report de mesure. Le protocole pour lequel cet objet est tenu dans un plan de même profondeur que celui des yeux est soumis aux erreurs de parallaxe, et n'offre en pratique aucune garantie que le décalage de profondeur entre les plans est faible. Les utilisateurs commettent en général une erreur non centrée de l'ordre du centimètre, ce qui est trop imprécis pour atteindre la qualité de mesure désirée pour le report. Le contact doit être un contact plan, c'est-à-dire que l'objet mire doit avoir au moins 3 points de contacts non alignés, pour assurer la stabilité du positionnement. La solution qui consiste, dans le cas du protocole précédent, à mettre une carte de crédit en contact sur la tranche au niveau des yeux, va nécessiter la rotation de la tête ainsi que la connaissance du calibrage pour retrouver à partir de plusieurs images la position et l'orientation de l'objet mire.

L'ensemble de ces contraintes favorise une mire de taille limitée, positionnée autour de la grandeur à mesurer. La mire idéale est une paire de lunettes de taille connue sur la face, et de distance face-œil connue. Cette mire permet un placement idéal en position, et aide à une orientation de face par projection symétrique de la surface visible des branches dans l'image. Un autre type de mire qui correspond est une carte de crédit ou un CD, que l'on situe préférentiellement sur la zone bouche-menton ou sur le front.

### Relation point visé/caméra/utilisateur

Le système des iris est non rigide. On considère les centres des globes oculaires comme centres de rotation de l'œil. Sont visibles depuis la camera l'iris et la pupille.

◦ Visée : lors des protocoles habituels de mesure réalisés dans les magasins d'optique, l'utilisateur regarde à l'infini afin de mesurer la mesure distance pupillaire à l'infini. On considère que la mesure du regard à l'infini peut être faite quand le sujet regarde à un point situé à plus d'une distance seuil *'ds'* (généralement un mètre, mais on peut descendre à 70cm et augmenter à l'infini), dans le cadre d'un report de mesure sur une image.
Les deux protocoles possibles dans le cadre de notre système sont les suivants :

1. L'utilisateur doit viser un point situé derrière la caméra à plus de la distance seuil ds.
2. L'utilisateur doit se situer à plus de la distance seuil ds et regarder la caméra ou un point proche.

◦ Convergence : la vision de l'utilisateur est plus ou moins précise, et sa capacité de convergence sur le point à viser peut varier. Dans le cas général, l'utilisateur ayant un problème de vue, sa capacité à viser correctement est limitée. Le fait qu'il soit à un peu moins d'un mètre du point de visée ne perturbe pas précision de la mesure. Pour un utilisateur qui voit bien, sa capacité à bien converger va imposer de se tenir loin pour respecter les conditions de visée à l'infini, qui permettent la mesure 2D.

Plus l'utilisateur est loin de la caméra, moins l'erreur de convergence ou visée sera importante.

### Précision de l'image et indices de mesure

Une autre source d'erreur est la précision de l'image. Afin de pouvoir effectuer un report de mesure, il faut repérer sur l'image obtenue des amers, qui permettent d'effectuer le report. En général, les systèmes proposés demandent de spécifier une longueur connue sur l'objet mire et le centre des pupilles sur les yeux. Le problème alors rencontré et non traité par les systèmes existants et l'erreur de pointage et de repérage de ces amers, qui est faite par un humain le plus souvent, ou un algorithme de reconnaissance, mais dont l'imprécision est telle que c'est une source d'erreur plus importante que l'erreur de parallaxe décrite plus haut pour des capteurs classiques.
D'une part, le système de numérisation de la scène réelle à l'image numérique finale additionne un nombre de traitements classiques inhérents aux capteurs numériques grand public : quantification, compression [autres]. La qualité des capteurs augmente avec le temps, mais la qualité de l'image n'est pas toujours bonne à cause de la combinaison de plusieurs facteurs connus comme la sensibilité du capteur, les conditions lumineuses, le temps de transfert matériel de la donnée numérique qui impose des contraintes de taille et de compression. L'image finale est souvent bruitée, saturée ou manque de dynamique, ce qui a pour effet de limiter la précision de la localisation des amers. Les capteurs peuvent avoir des résolutions élevées, les algorithmes de compression apportent beaucoup d'imprécision sur les contours ou en hallucinent de nouveaux. Sur un capteur HD webcam le contour d'un objet plan peut faire plusieurs pixels de large.
D'autre part, le repérage d'amers stables entre les images est difficile à cause de l'imprécision de l'image. Les systèmes actuels laissent l'utilisateur référencer ces amers, qui ne sont pas des opérateurs experts. L'erreur commise sur le pointage de l'amer désiré est très grande. Les algorithmes de détection n'étant jamais fiables à 100%, particulièrement pour des données très bruitées, les systèmes automatiques peuvent obtenir des erreurs aussi grossières qu'un humain inexpérimenté. La précision de pointage de l'amer étant perturbée par la visibilité et le pointage lui-même, les erreurs s'additionnent et on peut atteindre des erreurs de plusieurs pixels.
Sur les images de capteurs grand public, et avec les contraintes de placement de l'utilisateur, ces pixels d'erreur représentent plusieurs millimètres, ce qui rend la précision de la mesure de la distance pupillaire insuffisante.

**Solutions générales pour l'analyse. Protocole et outils pour garantir les contraintes**

Nous déduisons de l'analyse précédente les protocoles optimaux en fonction des types de capteur grand public :

**Avec une seule image :**

- Appareil photo: Avec des bons capteurs, comme des appareils photos, le protocole idéal est de se positionner de face et à plus de deux mètres. Il faut alors deux personnes (une pour prendre la photo), ou disposer d'un déclencheur automatique. En pratique, la position et l'orientation du système UM ne sont pas garantis : il faut que les deux personnes aient la même taille pour éviter les erreurs de parallaxe dus à la position, et obtenir une image pour laquelle l'orientation du visage est faible. Il faut donc réaliser un alignement des positions et orientations respectives des systèmes UM et caméra. Un protocole possible est que les deux personnes soient assises sur des chaises, afin de limiter leur différence de taille, à une distance la plus éloignée telle que l'utilisateur peut encore regarder l'objectif. Deux mètres sont suffisants en pratique. L'alignement des orientations entre les systèmes UM et caméra peut être faite avec les recommandations de positionnement de l'utilisateur proposées dans ce brevet. Comme le système de prise de vue n'a pas d'écran, les indications de positionnement ne sont pas effectuées par le logiciel mais par la personne qui prend la photo.
- Webcam : la webcam, intégrée ou non, est positionnée en général au-dessus de l'écran. Mêmes pour les capteurs de qualité, la compression de l'image est souvent forte, ainsi que la présence de traitements de l'image qui modifient les contours, et le réglage des paramètres de luminosité, balances des blancs et autres paramètres souvent adaptés de manière automatique très influents. Ces effets perturbent la position perçue des amers. La webcam se règle pour une lumière neutre. L'utilisateur se place assis face à la caméra. Le programme qui contrôle la caméra prend la photo automatiquement. L'utilisateur devant être le plus loin possible, il faut trouver le meilleur compromis entre taille dans l'image qui doit être maximale et distance à la caméra. L'utilisateur doit se placer environ à une distance d'un bras, entre 70cm et 1m. Le programme lui retourne en temps réel l'image du flux acquis sur l'écran, et l'image est zoomée et contient un guide ovale en son centre tel que l'utilisateur se place dedans. Ainsi, les contraintes de centrage et position sont respectées.

Un guide sur la visibilité des deux oreilles pour l'angle d'orientation gauche-droite, ainsi qu'un guide d'alignement des yeux horizontal avec l'axe du dessus des oreilles (endroit où les branches sont posées) pour l'orientation haut-bas sont affichés.

Une variante à l'affichage d'un guide est de mettre en place un système de suivi de visage en 3D qui permet de prendre la photo optimale (meilleure pose 3D du visage) en faisant des retours temps réel sur l'orientation du visage de l'utilisateur.

• Capteur mobile (smartphone tablette) : les mêmes guides sont affichés grâce à un programme web ou une application. L'utilisateur peut se prendre seul avec la camera frontale qui lui renvoie son image, comme dans le cas de la webcam, ou peut être assistée d'une personne qui suit les guides pour permettre un bonne visée et réalisation du protocole, comme dans le cas ce l'appareil photo. Le cas où l'utilisateur se prend seul est propice aux erreurs de réalisation du protocole, car toutes les contraintes que nous mettons à jour n'ont pas la garantie d'être respectées. La variante décrite précédemment s'applique aussi dans ce cas.

Dans ces 3 cas décrits, une variante à l'utilisation d'une mire plane est possible en utilisant le modèle 3D métrique du visage (ou d'une partie du visage) de l'utilisateur (possibilité de scanner son visage au préalable, possibilité d'uploader son modèle 3D dans l'application). Après un recalage entre des points 2D du visage issus de l'image et de leurs correspondants sur le modèle 3D métrique, la mire sera alors constituée d'au moins 2 points 3D particuliers du modèle 3D de visage. Selon la densité du maillage 3D de visage de l'utilisateur, celui pourra alors aussi servir au suivi de visage proposé dans la variante précédente. En résumé, la connaissance du visage 3D métrique de l'utilisateur permet de faire un suivi de visage et le report de mesure sur des amers du visage 3D.

• Position et orientation du système UM

A partir de quelques images en nombre faible (inférieur à cinq), on considère que les yeux regardent un point à l'infini et que la direction du regard est alignée avec celle du système UM, c'est-à-dire dans une direction quasi perpendiculaire au plan de la mire plane. Si on considère que les yeux bougent et suivent un point indépendamment de l'orientation du visage, alors on revient dans le cas du brevet précédent FR 10 52001, avec résolution 3D calibrée nécessaire. On examine le cas pour lequel l'orientation de la caméra et du système UM ne sont pas alignés.

◦ CAS 1 : système des yeux dans le même plan que le plan mire, un point de vue.

Si on considère le système des yeux dans un même plan ou un plan proche que celui de la mire, alors, pour une mire plane, on peut définir une relation homographique entre un espace de référence métrique et l'image. Cette relation peut s'écrire :

xref_i=H* x_i, avec **x_i** des points homogènes dans **P3,** espace projectif de dimension 3.

Il est alors possible d'effectuer un report de mesure non plus directement sur l'image entre les points x_i, mais dans un espace de l'image normalisée qui garantit la conservation des distances, entre les points xref_i, définis comme les images des x_i par une homographie **H.** La mise en oeuvre de cette mesure corrigée peut s'effectuer à partir d'une seule vue ou de plusieurs images, à des position et orientation de UM différentes.

Cette approche permet de minimiser les erreurs de précision du référencement des points d'intérêt dans les images.

CAS 2 : système des yeux dans des plans différents que celui de la mire, un point de vue

On ne peut pas effectuer de rapport de mesure fiable dans ce cas général à partir d'une seule image, à part dans le cas ou l'orientation est faible et la distance UM à la caméra est grande, ou les erreurs sont négligeables devant l'échelle de la mesure.

**Avec plusieurs images :**

- 1/ Si la caméra n'est pas calibrée, les scénarios décrits précédemment peuvent être utilisés sur plusieurs images ce qui permet de rendre la mesure robuste en s'intéressant à la statistique des mesures (comme par exemple la moyenne ou l'écart type).

- 2/ caméra en mouvement et utilisateur qui regarde un point à l'infini sans bouger. L'utilisateur qui réalise la prise de vue n'a besoin d'effectuer qu'un petit déplacement. La mire doit être visible dans le mouvement et positionnée de manière rigide par rapport au visage. A partir d'un nombre grand d'images acquises (deux en théorie), ou d'une vidéo, on peut reconstruire en 3D par des techniques classiques le système rigide UM de façon métrique dans le cas où la caméra est calibrée.

Une variante est de remplacer la mire plane par l'utilisation d'un modèle 3D métrique du visage (ou d'une partie du visage) de l'utilisateur (possibilité de scanner son visage au préalable, possibilité d'uploader son modèle 3D dans l'application).

Si la caméra n'est pas calibrée, il n'est pas possible d'estimer la relation métrique entre la mire et les yeux, et on se ramène au cas où il faut la définir (même plan ou non, relation rigide 3D, etc.). Le problème pratique qui se pose est alors celui de la précision numérique de cette relation, qui dépend de la résolution d'image, de la distance et de la taille de l'objet mire par rapport à la précision requise sur la mesure à effectuer. En général, pour une précision de l'ordre de 0.5 millimètres sur la distance pupillaire, la précision requise de la relation à définir entre caméra et yeux **CY** est du même ordre, ce qui est impossible à obtenir dans la réalité.

Prenons l'exemple que l'on connaît la taille et la morphologie 3D du visage de l'utilisateur, le visage constitue pour les yeux la meilleure mire que l'on peut obtenir. Si l'on veut mesurer la distance entre les centres des pupilles, alors il faut déterminer la relation de la profondeur du plan des pupilles par rapport à ce visage, et une erreur de l'ordre de demi-millimètre apporte une imprécision du même ordre.

Enfin, étant dans un contexte non calibré, la résolution avec relation homographique et une seule image est suffisante, puisqu'elle permet le report de mesure dans un espace normalisé. L'approche 3D ici décrite amène cependant une stabilité sur la mesure, puisque le nombre d'images et l'orientation de l'utilisateur qui réalise la prise de vue avec la caméra permettent d'obtenir une statistique de la mesure, et de caractériser ainsi la loi de l'erreur commise. Une manière de procéder est de reconstruire le système Yeux/Mire de la manière suivante (voir figure 1):

A / Trouver tout d'abord la transformation (Translation, Rotation) subit par l'ensemble des points de la mire dans chaque image acquise.

Pour chaque image, une minimisation par différence de points est réalisée (minimisation classiquement résolue par des algorithmes de type Gauss-Newton). Nous cherchons à minimiser l'expression suivante :

$$argmin_{Rm,Tm} \sum_{i=1}^{nPts} [P_{2D}(i) - Proj(Dp(P_{3D}(i), Rm, Tm)]^2$$

avec

**Rm,** matrice de rotation 3D entre caméra et mire,

*Tm,* vecteur translation 3D entre caméra et mire,,

*nPts,* nombre de points projetés,

$P_{3D}$, coordonnées 3D de la mire,

$P_{2D}$, coordonnées 2D de la mire dans l'image (coins, points contours, points caractéristiques),

*Proj,* fonction projetant un point 3D dans l'image (modèle sténopé par exemple),

$$\text{Proj}(P_{3D}) = \begin{bmatrix} x/z \\ y/z \end{bmatrix}$$

où

$$\begin{bmatrix} x \\ y \\ z \end{bmatrix} = KM * P_{3D}$$

et **KM,** matrice de calibrage de la caméra (précisions dans la partie calibrage)

$$KM = \begin{bmatrix} f & 0 & u0 \\ 0 & f & v0 \\ 0 & 0 & 1 \end{bmatrix}$$

$D_p$, étant la fonction appliquant la matrice de rotation R et le vecteur de translation **T** à un point 3D pour un déplacement en 3D

$$D_p(P_{3D}) = R* P_{3D} + T.$$

B/ Trouver ensuite la valeur de chaque variable $T_{SM}$, $R_{SM}$ et *hpd* communes à l'ensemble des images acquises.

Avec :

*hpd,* distance entre le centre du système des yeux et le centre de chaque pupille,

*Rsm,* matrice de rotation 3D rigide entre la mire et le système des yeux,

*Tsm,* vecteur translation 3D rigide entre la mire et le système des yeux,

Sur l'ensemble des images, minimisation par différence de points

$$\underset{\substack{hpd, \\ Rsm, Tsm}}{\text{argmin}} \sum_{i=1}^{nPts} \sum_{j=1}^{nIm} \left[ \begin{matrix} P'_{2D}(i,j) - \\ Proj(Dp(Dp(P'_{3D}(i,hpd),Rsm,Tsm),Rm(j),Tm(j))) \end{matrix} \right]^2$$

Avec :

*Rm,* matrice de rotation 3D de la mire (par image),

*Tm,* vecteur translation 3D de la mire (par image),

*nPts,* nombre de points projetés,

*nIm,* nombre d'images acquises,

$P'_{3D}$, coordonnées 3D des pupilles,

$P'_{2D}$, coordonnées 2D des pupilles dans l'image,

*Proj,* fonction projetant un point 3D dans l'image (par exemple par modèle sténopé),

$D_p$, fonction appliquant une matrice de rotation R et un vecteur de translation *T* à un point 3D pour un déplacement en 3D.

- 3/ Caméra fixe et utilisateur en mouvement qui regarde un point à l'infini. La mire doit être visible dans le mouvement et positionnée de manière rigide par rapport au visage. A partir d'un nombre grand d'images acquises, deux en théorie), ou d'une vidéo, on peut reconstruire en 3D par des techniques classiques le système rigide UM de façon métrique dans le cas où la caméra est calibrée.

Une variante est de remplacer la mire plane par l'utilisation d'un modèle 3D métrique du visage.

- 4/ Caméra calibrée fixe et utilisateur en mouvement qui regarde plusieurs points sur un écran/plan.
   Une variante est de remplacer la mire plane par l'utilisation d'un modèle 3D métrique du visage.
- 5/ Caméra calibrée en mouvement et utilisateur fixe qui regarde plusieurs points sur un écran/plan.
   Une variante est de remplacer la mire plane par l'utilisation d'un modèle 3D métrique du visage
- 6/ Caméra non calibrée fournissant une carte de profondeur (de type Kinect -marque déposée-). Un report de mesure peut être réalisé de manière plus précise puisque dans ce cas, la distance utilisateur caméra et caméra-mire sont connues. Elles ne sont plus simplement estimées comme dans les protocoles avec une seule image.
- 7/ Caméra calibrée fournissant une carte de profondeur (de type Kinect -marque déposée-). Dans ce cas aucune mire n'est nécessaire. Connaissant le calibrage de la caméra ainsi que la profondeur du visage dans la scène, on en déduit directement la distance entre les pupilles.

**Solution proposée de report simple avec précision <0.5mm pour un capteur grand public sans calibrage**

**Architecture du système**

- Une caméra : webcam, appareil photo, tablette, caméra carte de profondeur,
- Un calculateur, un écran,
- Un protocole précis de positionnement Utilisateur, Mire, Caméra U, M, C,
- Un ensemble de consignes interactives qui permet de respecter ce positionnement sans erreur,
- Un outil de référencement avec consignes des amers,
- Un calcul de report de mesure corrigé.

Des mires aux propriétés suivantes :

- Surface de contact suffisante pour assurer une stabilité sur le visage
- Autour des yeux, ou le plus proche possible : centré et près des éléments dont on veut effectuer les mesures.
- Des objets qui contiennent une certaine symétrie pour un positionnement aisé.
- Exemples de mire :

   ◦ des lunettes de dimensions et distance face-œil connue (en général 15mm).
   ◦ Une carte de crédit
   ◦ Un CD
   ◦ Un visage de dimensions connues, ou d'éléments constitutifs mesurés (comme les coins des yeux)

**Protocole et indications de réalisation**

Le sujet doit se placer à une distance connue (ou estimable) du dispositif d'acquisition d'image et regarder devant lui (un point particulier ou le dispositif d'acquisition par exemple) en plaçant un objet rigide de taille connue (mire) à une distance connue (ou estimable) du plan contenant les pupilles. Il est rappelé que dans le cas où le visage 3D métrique de l'utilisateur est connu, la mire plane n'est pas nécessaire.

Une acquisition d'image du sujet est alors réalisée lorsque sa tête est bien droite face à la caméra (oreilles et yeux quasiment alignés). Idéalement, les 2 oreilles du sujet doivent être visibles de manière équivalente. Un flux vidéo peut être enregistré pendant que l'utilisateur effectue un mouvement particulier de la tête permettant de choisir l'image la plus appropriée. L'image peut aussi être automatiquement choisie grâce à un suivi de visage 3D.

Le placement idéal de l'objet rigide se situe à une distance fixe ou estimable (statistiquement) du plan contenant les pupilles, une face de l'objet mire devant se trouver le plus parallèle possible à ce plan contenant les pupilles. Par exemple, l'objet rigide peut se trouver sur le front, sur une joue ou sur la bouche.

Les indications pratiques sont les suivantes dans le cas où la mire est une carte de crédit:

1. L'utilisateur se positionne assis devant son écran, à une distance d'un bras tendu de la caméra, supposée située en haut de l'écran et centrée. Il doit voir son visage centré dans l'écran.
2. S'il est porteur de lunettes, il les retire. Pour la majorité des corrections, notre protocole permet de garantir la qualité de la mesure sans avoir besoin de retirer les lunettes.

3. Il positionne la carte sur sa bouche, en veillant à exercer une pression suffisante avec son index pour que la carte soit fixée et respecte la condition de stabilité plane.

4. L'utilisateur règle ensuite l'orientation de sa tête en veillant à ce que

   a. En hauteur : la ligne horizontale passant par ses pupilles, ou les coins de ses yeux, soit de même hauteur dans l'image que les points de début d'oreilles au niveau des temps, soit les points sur lesquels reposeraient des branches de lunettes.

   b. En largeur : garantir que l'utilisateur voit les parties gauche et droite de son visage de manière symétrique, grâce à des indices. En positionnement, cela a été traité au point 1. En orientation, cette garantie s'effectue en donnant à l'utilisateur des repères sur l'image qui garantissent une orientation de face sans avoir tourné la tête trop à gauche ou à droite. Par exemple, les deux oreilles doivent être visibles. Les oreilles étant dans un plan éloigné d'au moins 10cm derrière les yeux, tout angle même faible de la tête entraîne un déplacement conséquent de ces objets dans l'image, et une invisibilité due à l'occultation provoquée par la tête depuis le point de vue de la caméra. Les oreilles disparaissent très vite si la tête n'est pas alignée en direction avec la visée de la caméra.

5. L'utilisateur regarde la camera

6. Le logiciel prend la photo

Une variante :

1. L'utilisateur se positionne assis devant son écran, à une distance d'un bras tendu de la caméra, supposée située en haut de l'écran et centrée. Il doit voir son visage centré dans l'écran.

2. S'il est porteur de lunettes, il les retire. L'utilisateur tient la carte à sa disposition.

3. Détection et suivi de visage 3D.

4. Le suivi de visage aide l'utilisateur à bien positionner son visage.

5. L'utilisateur porte la carte sur sa bouche et regarde la caméra.

6. Le logiciel prend la photo.

Une autre variante (dans le cas où le visage métrique est connu):

1. L'utilisateur se positionne assis devant son écran, à une distance d'un bras tendu de la caméra, supposée située en haut de l'écran et centrée. Il doit voir son visage centré dans l'écran.

2. S'il est porteur de lunettes, il les retire.

3. Détection et suivi de visage 3D métrique.

4. Le suivi de visage aide l'utilisateur à bien positionner son visage.

5. L'utilisateur regarde la caméra.

6. Le logiciel prend la photo.

Les indications et retours interactifs sont les suivants :

1. Positionnement : une image du positionnement. Un guide symétrique (rectangulaire, ovale) centré dans l'image dont la taille dépend de la résolution de la caméra et d'une longueur focale supposée. Ce cadre permet à l'utilisateur de valider son positionnement en déplacement et profondeur par rapport à la caméra. Des lignes verticales ou horizontales peuvent être ajoutées pour aider à la hauteur du positionnement des yeux ou à la symétrie horizontale du visage.

2. Une indication pour retirer ses lunettes

3. Un ou plusieurs exemples de positionnement

4. Des guide facultatifs pour la prise de vue : u

   a. En hauteur : une série de 3 lignes parallèles horizontales indiquant la ligne des yeux sur laquelle se positionner et la variabilité autorisée au-dessus ou au-dessous, correspondant à une rotation verticale de la tête vers le haut ou le bas, qui permet la précision attendue de la mesure.

   b. En largeur : des indications visuelles sur la visibilité.

**Calcul du report de mesure entre la mire et la distance oculaire à mesurer**

La résolution de la mesure consiste à faire un report de mesure entre la taille de l'objet mire et la taille en pixels de la grandeur à mesurer sur l'image acquise. En effet, en considérant l'objet mire et la grandeur à mesurer dans le même plan, la relation entre le nombre de pixels définissant une grandeur de l'objet mire et sa taille réelle sert de référence pour calculer D, la distance entre:

- les centres des pupilles, dans le cas du calcul du *PD.*
- le centre d'une pupille et l'arête nasale du sujet (ou le centre de la monture), dans le cas du calcul du *monoPD.*
- le bas de la monture d'une paire de lunettes et le centre d'une pupille, dans le cas du calcul de la hauteur.

La mesure *D* en mm est alors estimée par $D = dP * tMM / tMP$. Avec **dP,** la distance D exprimée en pixels (dans l'image), **tMM** la grandeur mesurable de l'objet rigide (mire) en mm et **tMP,** la taille de la mire en pixels sur l'image acquise (voir.figure 1 et figure 2).

Pour plus de précision, des correctifs d'amélioration peuvent être apportés :

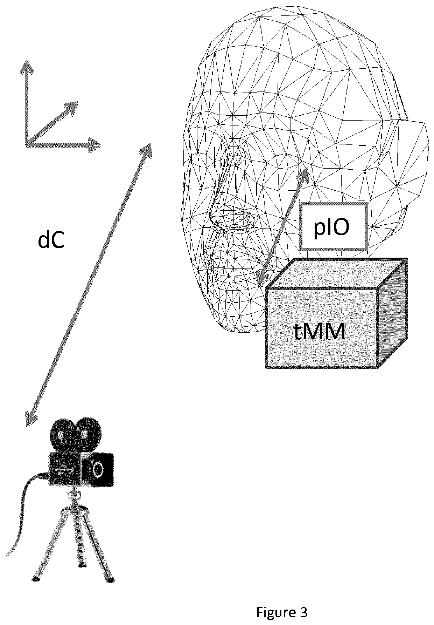

Si la distance focale **(fe)** du dispositif d'acquisition est inconnue, elle peut être estimée par $fe = dC * tMP / tMM$. Avec **dC,** la distance (connue, estimée ou supposée) entre le sujet et le dispositif d'acquisition en millimètres (voir figure 3).

La taille de la mire en pixels sur l'image acquise peut alors être corrigée (la valeur corrigée est notée **tMP2**), il s'agit d'une estimation de la taille que l'objet mire aurait s'il se trouvait dans le plan des pupilles (dans le cas du calcul de PD et *monoPD*) ou des verres (dans le cas du calcul des hauteurs) :

$tMP2 = tMP * fe / (dC + p/O)$. Avec **pIO,** la profondeur (connue, estimée ou supposée) entre le plan contenant la grandeur connue de l'objet rigide (mire) et le plan contenant les pupilles (dans le cas du calcul de PD et *monoPD*) ou les verres (dans le cas du calcul des hauteurs).

La mesure D corrigée vaut : $dP * tMM / tMP2$.

Enfin, si la distance *dC* est inférieure à 60 cm et que le regard du sujet ne se porte pas à plus de 1 m, un correctif peut être ajouté pour passer de la distance pupillaire de près à celle de loin.

En pratique, un logiciel permettant l'acquisition et les calculs peut prendre la forme d'une application et/ou d'un site internet/plugin pour navigateur. Ce logiciel peut guider l'utilisateur quant au placement de sa caméra et à son positionnement du regard.

Dans le cas où le calibrage de la camera est connu, il est évident que l'estimation de la focale (fe) n'est plus nécessaire, la précision de la mesure est donc améliorée.

## Outil de traitement pour la précision des indices image

Afin de permettre une grande précision de référencement des indices, nous proposons une interface adaptée. Cette interface peut être utilisée par l'utilisateur final, mais est surtout prévue pour être utilisée par un expert formé afin d'assurer une stabilité de traitement.

L'expert utilise cette interface pour :

1. repérer les erreurs de positionnement et d'orientation.
2. Choisir la meilleure photo selon des critères de qualité de réalisation de protocole, mais aussi de visibilité des indices. L'expert peut refuser des photos de qualité non suffisante pour repérer les indices ou qui montrent une réalisation de protocole insuffisante. L'architecture du système permet alors de recontacter l'utilisateur afin qu'il réalise une prise de vue supplémentaire dans de meilleures conditions.
3. référencer les indices image qui permettent la mesure.

## Fonctionnalités de l'interface revendiquées

Afin de garantir le maximum de précision lors du placement des amers, l'interface garantit à tout moment et dans la même visualisation :

1. une vue globale de la scène
2. une vue zoomée locale de la partie de l'image ou l'indice est à référencer.
3. Des guides et indications de positionnement pour les cas difficiles

Ces vues couplées permettent à l'utilisateur expert d'être précis et de parfaire son apprentissage du référencement.

Lors de la présentation de l'image à référencer, celle-ci a déjà été zoomée sur les parties intéressantes pour la vue globale, grâce au positionnement de l'utilisateur maîtrisé lors du protocole.

*Référencement des amers des yeux*

Afin de référencer les centres des yeux, nous proposons le placement d'un cercle muni de lignes de visées pointant vers le centre du cercle sur l'iris par l'utilisateur expert. Le placement doit être effectué tel que la courbure du cercle suive celle de l'iris à égale distance euclidienne en chaque point. Lors de ce placement, la partie zoomée de l'interface permet d'être très précis.

*Référencement des amers de la mire*

Pour une carte de crédit, l'interface propose une approche globale et locale au niveau de la manipulation de la forme de référencement. Un rectangle est présenté à l'utilisateur, de taille et de positions proches de celles attendues sur l'image. Ce rectangle possède quatre points de contrôle. Un mode global de déformation permet de gérer une déformation en échelle et rotation, avec un ratio fixé largeur/hauteur. Normalement, la carte bien positionnée lors de la réalisation du protocole doit présenter une image qui permet d'aligner le modèle géométrique de l'interface avec l'image.

La partie zoomée de l'interface permet de gérer les incertitudes locales dues aux problèmes de compression et quantification de l'image. Le deuxième mode de modification de la forme géométrique de la carte permet de modifier la position des points un à un et indépendamment des autres. Ces points étant reliées entre eux, l'utilisateur expert peut visualiser la ligne formée entre ces points et ainsi suivre la silhouette de la carte exactement. La forme géométrique épouse alors les contours de la déformation projective de l'image la carte réelle. Si cette déformation est trop importante, cela donne une indication sur la qualité de réalisation du protocole. Souvent, les problèmes de compression de l'image et de quantification inadaptée surviennent dans les zones de faible contraste. Ainsi, les contours ne sont plus visibles tout au long des bords. L'utilisateur expert peut alors choisir les zones les plus pertinentes sur lesquelles s'appuyer. Une bande noire étant présente au dos de la carte, le contraste peut être fort à ce niveau, et le rectangle géométrique peut être adapté sur cette bande si nécessaire.

On a une précision locale compensée par une cohérence globale pour le référencement. Enfin, des algorithmes d'apprentissage peuvent être mis en place pour garantir ces contraintes lors d'un ajustement de modèle géométrique sur l'image exemple (contour, texture, etc.).

La distance utilisée pour la mesure, est la distance située au niveau du haut de la bande, qui correspond aux points d'appuis les plus stables de la carte sur le visage, et pour lesquelles les hypothèses émises pour le report sont les plus fiables.

Pour tout autre objet plan, on veillera à respecter la possibilité de déformations projectives sur la silhouette de l'objet considéré, afin de coller au maximum à sa silhouette sur l'image. Ainsi, pour un CD, on ajustera un double cercle qui pourra se transformer en ellipse.

Ces référencements sont valides autant pour le protocole avec personne de face que celui avec personne orientée et utilisation d'un espace normalisé par relation homographique.

**Extensions**

Système multi-caméra calibré : reconstruction 3D

1. Rien ne bouge, regard à l'infini
2. Les yeux regardent un point qui se déplace sur un écran

Le procédé ici décrit, garantit une réussite de la mesure grâce à :

- un protocole facile à comprendre et à réaliser,
- des guides interactifs
- un outil d'annotation facile à comprendre et utiliser qui garantit la précision de l'annotation des amers nécessaires à la mesure.

Le procédé s'affranchit des erreurs de parallaxe et de détection, et garantit les bonnes conditions et corrections pour un report de mesure juste.

**Revendications**

1. Procédé de détermination d'au moins une mesure oculaire d'un utilisateur à partir d'au moins une image de la tête de l'utilisateur acquise par un capteur d'images numériques, de type grand public, la mesure oculaire étant comprise parmi la distance pupillaire, la distance mono pupillaire ou les hauteurs, l'utilisateur voyant l'image de sa tête s'afficher sur un écran, ledit procédé comprenant les étapes suivantes :

a. guidage de l'orientation tridimensionnelle de la tête de l'utilisateur par rapport au capteur d'images en superposant des repères visuels sur l'image comprenant la tête de l'utilisateur s'affichant sur l'écran, les repères comprenant un guide symétrique centré dans l'image pour le positionnement en déplacement et en profondeur par rapport à la caméra, un guide de positionnement horizontal des yeux et un guide de symétrie horizontale du visage;

b. calcul de la mesure oculaire à partir de la mesure en pixels d'une dimension connue d'un objet compris dans l'image, l'objet ayant une dimension connue, et à partir de la dimension en pixels de la mesure oculaire dans l'image ; l'étape de guidage de l'orientation comprenant une sous-étape de suivi en temps réel de la position et de l'orientation tridimensionnelle du visage de l'utilisateur, les repères visuels étant déterminés en fonction de la position et de l'orientation du visage de l'utilisateur à chaque instant.

2. Procédé selon la revendication 1, **caractérisé en ce que** l'objet ayant une dimension connue est un objet plan.

3. Procédé selon l'une quelconque des revendications 1 à 2, **caractérisé en ce que** l'objet plan est appuyé contre le visage de l'utilisateur, l'objet étant centré sur le plan sagittal de la tête de l'utilisateur.

4. Procédé selon la revendication 1, **caractérisé en ce que** l'objet ayant une dimension connue est une paire de lunettes.

5. Procédé selon la revendication 1, **caractérisé en ce que** l'objet ayant une dimension connue est le visage de l'utilisateur.

6. Procédé selon l'une quelconque des revendications 1 à 5, **caractérisé en ce que** l'image de l'individu comprend une vue de face de la tête de l'individu.

7. Procédé selon l'une quelconque des revendications 1 à 6, **caractérisé en ce que** le capteur grand public acquiert une image calibrée en dimension en se déplaçant autour du visage de l'utilisateur qui regarde un point à l'infini.

8. Procédé selon l'une quelconque des revendications 1 à 6, **caractérisé en ce que** le capteur grand public acquiert une image calibrée en dimension en se déplaçant autour du visage de l'utilisateur qui regarde des points affichés sur un écran.

9. Procédé selon l'une quelconque des revendications 1 à 8, **caractérisé en ce que** le capteur grand public fournit une carte de profondeur de l'image acquise.

**Patentansprüche**

1. Verfahren zur Bestimmung von mindestens eines Maßes eines Auges eines Benutzers auf der Basis von mindestens einem Bild des Kopfes des Benutzers, das von einem digitalen Bildsensor vom Consumer-Typ aufgenommen wird, wobei das Maß eines Auges der Pupillenabstand, der Mono-Pupillenabstand oder die Höhen ist, wobei der Benutzer das Bild seines Kopfes auf einem Bildschirm angezeigt sieht, wobei das Verfahren die folgenden Schritte umfasst:

a. Führen der dreidimensionalen Ausrichtung des Kopfes des Benutzers im Verhältnis zum Bildsensor durch Übereinanderlagern visueller Bezugspunkte auf dem Bild, die den Kopf des Benutzers, der auf dem Bildschirm angezeigt ist, umfassen, wobei die Bezugspunkte eine im Bild zentrierte symmetrische Führung für die Bewegungs- und Tiefen-Positionierung im Verhältnis zur Kamera, eine horizontale Positionierungsführung der Augen und eine horizontale Symmetrieführung des Gesichts umfasst;

b. Berechnen des Maßes eines Auges auf der Basis der Messung in Pixeln einer bekannten Abmessung eines Objekts im Bild, wobei das Objekt eine bekannte Abmessung hat, und auf der Basis der Abmessung in Pixeln des Augenmaßes im Bild;

wobei der Schritt der Führung der Ausrichtung einen Unterschritt der Nachverfolgung in Echtzeit der Position und der dreidimensionalen Ausrichtung des Gesichts des Benutzers umfasst, wobei die visuellen Bezugspunkte in Abhängigkeit von der Position und von der Ausrichtung des Gesichts des Benutzers zu jedem Zeitpunkt bestimmt werden.

**2.** Verfahren nach Anspruch 1, **dadurch gekennzeichnet, dass** das Objekt, das eine bekannte Abmessung hat, ein ebenes Objekt ist.

**3.** Verfahren nach einem der Ansprüche 1 bis 2, **dadurch gekennzeichnet, dass** das ebene Objekt auf dem Gesicht des Benutzers abgestützt ist, wobei das Objekt auf der sagittalen Ebene des Kopfes des Benutzers zentriert ist.

**4.** Verfahren nach Anspruch 1, **dadurch gekennzeichnet, dass** das Objekt, das eine bekannte Abmessung hat, eine Brille ist.

**5.** Verfahren nach Anspruch 1, **dadurch gekennzeichnet, dass** das Objekt, das eine bekannte Abmessung hat, das Gesicht des Benutzers ist.

**6.** Verfahren nach einem der Ansprüche 1 bis 5, **dadurch gekennzeichnet, dass** das Bild des Individuums eine Vorderansicht des Kopfes des Individuums umfasst.

**7.** Verfahren nach einem der Ansprüche 1 bis 6, **dadurch gekennzeichnet, dass** der Consumer-Sensor ein abmessungsmäßig kalibriertes Bild aufnimmt, indem er sich um das Gesicht des Benutzers bewegt, der auf einen Punkt in Unendlich blickt.

**8.** Verfahren nach einem der Ansprüche 1 bis 6, **dadurch gekennzeichnet, dass** der Consumer-Sensor ein abmessungsmäßig kalibriertes Bild aufnimmt, indem er sich um das Gesicht des Benutzers bewegt, der auf Punkte blickt, die auf einem Bildschirm angezeigt sind.

**9.** Verfahren nach einem der Ansprüche 1 bis 8, **dadurch gekennzeichnet, dass** der Consumer-Sensor eine Tiefenkarte des erfassten Bildes bereitstellt.


**Claims**

**1.** Method for determining at least one ocular measurement of a user from at least one image of the user's head, acquired by a consumer-level digital image sensor, the ocular measurement being included among the pupillary distance, the monocular pupillary distance or the heights, the user viewing the image of their head displayed on a screen, said method comprising the following steps:

> a. guiding the three-dimensional orientation of the user's head relative to the image sensor by superimposing visual markers on the image comprising the user's head displayed on the screen, the markers comprising a symmetrical guide centred in the image for displacement and depth positioning relative to the camera, a guide for horizontal positioning of the eyes and a guide for horizontal symmetry of face;
> b. calculating the ocular measurement from the measurement, in pixels, of a known dimension of an object included in the image, the object having a known dimension, and from the dimension in pixels of the ocular measurement in the image;
> the step of guiding the orientation comprising a substep of monitoring, in real time, the position and the three-dimensional orientation of the user's face, the visual markers being determined as a function of the position and the orientation of the user's face at each instant.

**2.** Method according to claim 1, **characterised in that** the object having a known dimension is a flat object.

**3.** Method according to any one of claims 1 to 2, **characterised in that** the flat object is pressed against the user's face, the object being centred on the sagittal plane of the user's head.

**4.** Method according to claim 1, **characterised in that** the object having a known dimension is a pair of spectacles.

**5.** Method according to claim 1, **characterised in that** the object having a known dimension is the user's face.

**6.** Method according to any one of claims 1 to 5, **characterised in that** the image of the individual comprises a front view of the head of the individual.

**7.** Method according to any one of claims 1 to 6, **characterised in that** the consumer-level sensor acquires an image calibrated in dimension by moving around the user's face which is looking at a point at infinity.

8. Method according to any one of claims 1 to 6, **characterised in that** the consumer-level sensor acquires an image calibrated in dimension by moving around the user's face which is looking at points displayed on a screen.

9. Method according to any one of claims 1 to 8, **characterised in that** the consumer-level sensor provides a depth map of the acquired image.

Yeux

hpd

Tsm,Rsm

Mire

Tm,Rm

Caméra

Figure 1

Imag
e

dP

tMP

Figure 2

dC

pIO

tMM

Figure 3

**EP 2 999 393 B1**

## RÉFÉRENCES CITÉES DANS LA DESCRIPTION

**Documents brevets cités dans la description**

- US 6535223 B
- WO 201204154995 A
- FR 1052001